# EUROPÄISCHE PATENTANMELDUNG

(11) **EP 2 742 976 A1**
(43) Veröffentlichungstag der Anmeldung: **18.06.2014**
(21) Anmeldenummer: 13193033.1
(22) Anmeldetag: 15.11.2013
(51) Int. Cl.: A61Q 19/00, A61K 8/81

(54) **Milde, transparente schäumende Zubereitungen**

(30) Priorität: 12.12.2012 DE 102012222979
(71) Anmelder: Beiersdorf AG, 20253 Hamburg (DE)
(72) Erfinder: Argembeaux, Horst, 21465 Wentorf (DE); Quidde, Cornelia, 22453 Hamburg (DE); Counradi, Katrin, 22143 Hamburg (DE)

(57) **Zusammenfassung**

Transparente Reinigungssysteme mit stabil suspendierten Partikeln, enthaltend wenigstens ein Step-Polymer. Die Reinigungssysteme weisen geeignete Fließeigenschaften, einen pH-Wert von 4,5 bis 5,5 auf und sie zeichnen sich durch eine besondere Milde aus.

## Beschreibung

Die vorliegende Erfindung beschreibt transparente Reinigungssysteme, die stabil suspendierte Partikel enthalten. Dies wird erreicht durch eine Einarbeitung von einem oder mehreren Step-Polymeren in die Reinigungssysteme dergestalt, dass geeignete Fließeigeneigenschaften, charakterisiert durch eine Viskosität von 2000 mPa·s bis 5000 mPa·s, erreicht werden. Darüber hinaus weisen die erfindungsgemäßen Reinigungssysteme einen pH-Wert von 4,5 bis 5,5 auf und zeichnen sich durch eine besondere Milde aus.

Transparente Reinigungszubereitungen mit suspendierten oder stabilisierten Partikeln sind beim Anwender sehr erwünscht. Bisher ist es nicht möglich gewesen, klare Tensid-Formulierungen mit stabilisierten Partikeln bei einem pH-Wert von 4,5 bis 5,5 bereitzustellen, die geeignete Fließeigenschaften mit einer Viskosität von 2000 mPa·s bis 5000 mPa·s. und ein hohes Schaumvermögen sowie eine gute Milde aufweisen. Der Stand der Technik kennt zwar bereits Systeme mit Xanthangummi. Diese besitzen aber bezüglich des Hautgefühls während und nach der Anwendung kosmetische Eigenschaften, die verbessert werden können. Darüber hinaus werden bei gleicher Einsatzkonzentration nur geringere Viskositäten erreicht.

Um Fortschritte im Bereich der Stabilisierung gelartiger Systeme bei gleichzeitiger Verbesserung der Anwendungseigenschaften zu erzielen, wurden Versuche unternommen bei denen Xanthangummi durch polymere Strukturen ersetzt wurde. Besonders geeignet erwiesen sich vernetzte synthetische Polymere. Bei der Herstellung dieser Polymere spielen die Auswahl der Monomeren, das Verhältnis der Monomeren untereinander und der Ablauf der Polymerisationsreaktion eine wichtige Rolle. Besonders der Ablauf der Reaktion hat Einfluss auf die Eigenschaften der entstehenden Produkte. Die Polymerisationsreaktion kann in mindestens zwei Stufen durchgeführt werden. Die erste Stufe zeichnet sich dadurch aus, dass keine vernetzenden Monomere zugegen sind, so dass unverzweigte, polymere Strukturen entstehen. In einer zweiten Stufe des Reaktionsprozesses sind dann Vernetzer zugegen, dies führt zu einer Vernetzung der polymeren Strukturen. Das entstehende Produkt zeichnet sich durch einen inhomogenen Aufbau aus. Die Monomere, die in der ersten Reaktionsstufe eingesetzt werden, sind beispielsweise Monomere mit einer Carboxylgruppe (Acrylsäure, Methacrylsäure und ähnliche Verbindungen) und Monomere, die ein Alkylester oder Hydroxyalkylester mit 1 bis 5 Kohlenstoffatomen der Acrylsäure oder Methacrylsäure sind, optional können weitere Monomere mit α,β- ungesättigten Ethylengruppen zugefügt werden. In der zweiten Reaktionsstufe kommen neben den Monomeren, die bereits für die erste Reaktionsstufe eingesetzt wurden, Vernetzer zum Einsatz. Die chemischen Reaktionen der einzelnen Stufen werden vollständig durchgeführt, bevor die Reaktion der nächsten Stufe eingeleitet wird. Beispielhaft soll im Folgenden ein Syntheseprozess, der aus zwei Stufen besteht, beschrieben werden: In ein erstes Reaktionsgefäß wird unter einer inerten Atmosphäre zu einer wässrigen Emulgatorlösung (beispielsweise ein anionisches Tensid), eine Mischung aus Monomeren für die erste Stufe gegeben. Optional können weitere Hilfsstoffe zugegeben werden. Die entstehende Mischung wird gemischt, so dass eine Monomeremulsion entsteht. In ein zweites Reaktionsgefäß, ausgestattet mit einem Rührer, einem Zustrom für inertes Gas und Pumpen, werden unter einer inerten Atmosphäre Wasser, anionisches Tensid und optional weitere Hilfsstoffe gegeben. Unter Rühren wird diese Mischung auf 55 bis 98°C erhitzt, dann wird ein Reaktionsinitiator zugesetzt und ein Teil der Lösung aus dem ersten Reaktionsgefäß nach und nach, über einen Zeitraum von 30 Minuten bis 4 Stunden, in das zweite Gefäß gegeben. Die Reaktionstemperatur beträgt etwa 45 bis etwa 95°C. Nach Beenden der Monomerzugabe aus dem ersten Gefäß kann optional weiterer Reaktionsinitiator zugegeben werden. Die Reaktionstemperatur bleibt bei 45 bis 95°C bis die Polymerisationsrektion beendet ist. In das erste Reaktionsgefäß, das noch einen Teil der Emulsionsmischung mit den Monomeren enthält, wird Vernetzer zugegeben und emulgiert. Alternativ kann in einem weiteren Reaktionsgefäß die Mischung für die zweite Reaktionsphase gemischt werden. Die Monomeremulsion für die zweite Reaktionsphase wird mit einer konstanten Rate in das zweite Reaktionsgefäß, das die Reaktionsprodukte der ersten Stufe enthält, gegeben und gemischt. Erneut wird Reaktionsinitiator zugesetzt. Die Reaktionstemperatur beträgt etwa 85°C und die Reaktion läuft für 2,5 Stunden bis die Polymerisation beendet ist. Verbliebene Monomere können durch eine erneute Zugabe von Reaktionsinitiator eliminiert werden.

Die Produkte, die durch derartige Reaktionen entstehen, werden im Folgenden als Step-Polymere bezeichnet. In der WO 2012/006402 werden beispielsweise derartige Reaktionen und Produkte beschrieben. Zu den Step-Polymeren, die in diesem Dokument offenbart werden, zählt beispielsweise das Acrylate Crosspolymer-4. Das Acrylate Crosspolymer-4 ist beispielsweise erhältlich bei der Fa. Lubrizol unter dem Handelsnamen Carbopol® Aqua SF-2 Polymer.

In der vorliegenden Offenbarung werden die Begriffe Reinigungssystem, Reinigungszubereitung und Reinigungsformulierung oder Formulierung als Synonyme verwendet.

Es hat sich nun für den Fachmann überraschend und nicht vorhersehbar herausgestellt, dass die folgenden Zubereitungen den Nachteilen des Standes der Technik abhelfen, wobei die Zubereitungen transparente Reinigungszubereitungen
mit suspendierten Partikeln,
mit einem pH-Wert von 4,5 bis 5,5 und
mit einer Viskosität von 2000 mPa·s bis 5000 mPa·s sind,
dadurch gekennzeichnet, dass sie wenigstens ein Step-Polymer enthalten,
anionische, amphotere und optional nichtionische Tenside enthalten,
wobei der Gehalt an anionischen Tensiden unter 10 Gew.-%, bezogen auf die Gesamtmasse der Zubereitung, liegt.

Es ist erfindungsgemäß bevorzugt, wenn das Step-Polymer ein Acrylates Crosspolymer-4 ist.

Es ist erfindungsgemäß besonders bevorzugt, wenn die erfindungsgemäßen Zubereitungen L/D-Werte ≥ 0,5 aufweisen.

Auch erfindungsgemäß bevorzugt ist, dass die erfindungsgemäßen Zubereitungen frei von Xanthangummi sind.

Es ist erfindungsgemäß bevorzugt, wenn der Gehalt an wenigstens einem Step-Polymer höchstens 2,5 Gew.-%, bevorzugt höchstens 2,0 Gew.-%, insbesondere bevorzugt zwischen 1,0 und 1,6 Gew.-%, bezogen auf den Aktivgehalt des oder der Polymers/e und das Gesamtgewicht der Zubereitung, beträgt.
Erfindungsgemäß bevorzugt ist es ebenfalls, wenn der Gehalt an gelbildenden Polymeren einschließlich von wenigstens einem Step-Polymer höchstens 5,0 Gew.-%, bevorzugt höchstens 2,5 Gew.-%, insbesondere bevorzugt höchstens 1,6 Gew.-%, bezogen auf den Aktivgehalt der Polymere und das Gesamtgewicht der Zubereitung, beträgt.

Weiter besonders bevorzugt ist es, wenn das Step-Polymer neben den Monomeren mit einer Carboxylgruppe (Acrylsäure, Methacrylsäure und ähnliche Verbindungen) und Monomeren, die aus Alkylestern oder Hydroxyalkylestern mit 1 bis 5 Kohlenstoffatomen der Acrylsäure und/oder Methacrylsäure gebildet werden, zusätzlich höchstens 5 % weitere Monomere enthält. Die weiteren Monomeren können z.B. Vernetzer umfassen oder Monomere, die die polymeren Strukturen modifizieren. Optional denkbar sind Monomere enthaltend Ethoxygruppen.

Erfindungsgemäß besonders bevorzugt ist es, wenn der tan-delta der erfindungsgemäßen Zubereitungen kleiner 0,6 ist.

Weiter erfindungsgemäß besonders bevorzugt ist es, wenn die Transmission der oben beschriebenen Zubereitungen größer 80% ist.

Auch erfindungsgemäß ist ein Verfahren zur Herstellung der erfindungsgemäßen Zubereitungen, welches aus den folgenden Schritten besteht:
1. Wasser vorlegen (optional Farbstoff einarbeiten)
2. Polymer, einschließlich wenigstens eines Step-Polymers, in die Wasserphase geben,
3. anionisches Tensid zugeben
4. nicht-ionisches Tensid zugeben (falls in der Rezeptur vorgesehen)
5. amphoteres Tensid zugeben
6. Neutralisation mit NaOH auf einen pH-Wert von 6,5-6,8
7. Lichtschutzphase zugeben (falls in der Rezeptur vorgesehen)
8. Glycerin zugeben (falls in der Rezeptur vorgesehen)
9. Parfümphase zugeben (falls in der Rezeptur vorgesehen)
10. Konservierungsmittel-Phase zugeben (falls in der Rezeptur vorgesehen)
11. pH-Wert mit Citonensäure (50%ig) auf 4,5-5,5 einstellen
12. Partikel/Peelingkörper einarbeiten mit möglichst geringer Scherung.

Die Reihenfolge der Zugabe der jeweils einzelnen anionischen, nicht-ionischen und amphoteren Tenside in die Zubereitung, wie unter den Punkten 3,4,5 aufgelistet ist, ist erfindungserheblich. Die Zugabe in dieser Reihenfolge und Weise ermöglicht die Herstellung transparenter und stabiler Zubereitungen.

Für die Zubereitung einer erfindungsgemäß bevorzugten Lichtschutzphase (siehe Punkt 7) wird Benzophenone-4 in Wasser gelöst und entsprechend dem erfindungsgemäßen Herstellungsverfahren der Zubereitung zugesetzt.

Für die Zubereitung einer erfindungsgemäß bevorzugten Parfümphase wird eine Phase beispielsweise aus den Rohstoffen PEG-40 Hydrogenated Castor Oil, PEG-7 Glyceryl Cocoate, Parfuem und Helianthus Annuus Seed Oil hergestellt und entsprechend dem erfindungsgemäßen Herstellungsverfahren der Zubereitung zugesetzt.

Für die Zubereitung einer erfindungsgemäß bevorzugten Konservierungsmittelphase wird Sodium Benzoate/ Sodium Salicylate in Wasser gelöst und diese Phase wird entsprechend dem erfindungsgemäßen Herstellungsverfahren der Zubereitung zugesetzt.

Die Erfindung umfasst auch die Verwendung der erfindungsgemäßen Zubereitungen zur Reinigung der Haut des Menschen und/oder zur Reinigung der Haare des Menschen. Denkbar ist auch die Verwendung in anderen Gel-artigen Zubereitungen, die nicht der Reinigung der Haut und der Haare dienen, die jedoch auch die Eigenschaften der erfindungsgemäßen Zubereitungen, wie eine Viskosität von 2000 mPa·s bis 5000 mPa·s und einen pH-Wert von 4,5 bis 5,5 und optional eine Milde, beschrieben durch L/D-Werte ≥ 0,5 und optional tan-delta-Werte kleiner 0,6 und optional Transmissionswerte größer 80%, haben. Weiterhin denkbar ist auch die Verwendung von wenigstens einem Step-Polymer in kosmetischen Zubereitungen in erfindungsgemäßer Weise.

Die Viskositätswerte, die in der vorliegenden Schrift offenbart werden, sind mit dem Rheomat R123 der Gesellschaft ProRheo bei 25°C gemessen worden. Bei der Messung mit dem Rheomat R123 wird der Rotor des Gerätes blasenfrei bis zur Markierung in die Probe eingetaucht.

Die Transmission der Zubereitungen wurde durch VIS-Photometrie mit dem Specord 250 der Gesellschaft Analytik Jena bei 420nm in 1 cm Einmalküvetten gegen Wasser als Referenz (gemessen werden die Formulierungen ohne Farbstoff und Partikel) gemessen.

Durch die Einarbeitung von wenigstens einem Step-Polymer konnten transparente Formulierungen bei einem pH-Wert von <5,5 entwickelt werden. Transparente Formulierung bedeutet, dass die Transmission >80% ist. Die Messung erfolgt bei 420nm mit VIS-Photometrie in 1 cm Einmalküvetten gegen Wasser als Referenz, gemessen werden die Formulierungen ohne Farbstoff und Partikel.

Die erfindungsgemäßen Formulierungen weisen einen tan-delta von <0,6 auf, wodurch eine Stabilisierung von Partikeln gegeben ist. Unter dem tan-delta wird der Quotient aus dem Verlustmodul und dem Speichermodul verstanden. Der tan-delta wird wie folgt ermittelt:

Gemessen werden Verlust-und Speichermodul durch einen dynamischen Frequenztest auf einem schubspannungsgesteuerten Rheometer bei 40°C ± 1°C mit 25 mm Platte/Platte Geometrie bei einem Spalt zwischen 0,8 mm und 1,2 mm, wobei strukturschonend befüllt wird. Es wird nach dem Stand der Technik der Frequenztest mit einer entsprechenden Strukturerholungszeit vor dem Test durchgeführt und der tan-delta im Frequenzbereich zwischen 0,05 rad/s und 3,0 rad/s angegeben, bevorzugt zwischen 0,08 rad/s und 1,0 rad/s.

Aus dem Stand der Technik bekannte Rezepturen weisen einen anionischen Tensidanteil von >10% auf. Durch den hohen Anteil von anionischen Tensiden haben diese Rezepturen eine schlechte Milde (L/D-Wert <0,5).

Durch die erfindungsgemäße Art der Herstellung ist es gelungen klare Formulierungen mit einer Milde von L/D: ≥ 0,5 zu entwickeln. Bei der Art der Herstellung werden weniger als 10% anionische Tenside in Kombination mit amphoteren und/oder nichtionischen Tensiden eingesetzt. Die derart hergestellten Formulierungen weisen eine Transmission von > 80% auf und haben einen tan-delta von <0,6.

Für die Bestimmung der Milde wird ein RBC-Assay durchgeführt.

Der Standard-RBC-Assay (10 Minuten Inkubation) dient zur Abschätzung von in vivo Augenschleimhautreizpotentialen von Tensiden und tensidhaltigen Produkten.

### 1. Hämolyse

Ein definiertes Aliquot isolierter Kalbserythrozyten wird mit einer Reihe steigender Konzentrationen des zu untersuchenden WAS-Prüfmusters (Stammlösung mit Formulierungen 1:100 w/v bzw. für Rohstoffe 0.1 % Aktivgehalt in PBS) für 10 Minuten unter Schütteln bei Raumtemperatur (RT) inkubiert. Nach Zentrifugation werden die gewonnenen Überstände photometrisch auf ihren Gehalt an freigesetztem Hämoglobin (HbO₂) bei 530 nm analysiert. Daraus wird der relative Hämolysegrad berechnet und die Konzentrations-Response-Kurve mit dem H50-Wert [µl/ml] als Kenngröße bestimmt. Dieser gibt die Konzentration des Prüfmusters an, bei der 50% des Hämoglobins freigesetzt werden.

### 2. HbO₂-Denaturierung

Ein definiertes Aliquot isolierter Kalbserythrozyten wird mit einer fixen Konzentration des Prüfmusters (1% w/v bzw. 0.1% Aktivgehalt) für 10 Minuten unter Schütteln bei RT inkubiert und dann zentrifugiert. Die Änderung der spektralen Absorption bei 575 nm und 540 nm wird im Vergleich zum nativen HbO₂ gemessen. Aus dem Verhältnis der Absorptionswerte zueinander wird der Denaturierungsindex DI [%] berechnet. Als 100%-Standard dient Na-Laurylsulfat (0.1% Aktivgehalt).

### 3. L/D-Quotient

Der Quotient stellt das Verhältnis der Kenngrößen von Hämolyse (H50) und Denaturierung (DI) dar und wird zur Charakterisierung und Klassifizierung der untersuchten Prüfmuster verwendet.

Zur Verdickung der erfindungsgemäßen Zubereitungen und zur Stabilisierung von Partikeln in den erfindungsgemäßen Zubereitungen können optional weitere Polymere und Amphotenside eingesetzt werden, die ausgewählt werden können aus der Gruppe Siliciumdioxid, Polysaccharide bzw. deren Derivate, z. B. Hydroxypropylmethylcellulose, besonders vorteilhaft aus der Gruppe der Polyacrylate, bevorzugt ein Polyacrylat aus der Gruppe der so genannten Carbopole, beispielsweise Carbopole der Typen 980, 981, 1382, 2984, 5984, jeweils einzeln oder in Kombination, sowie alkoxylierte Ester wie z.B. PEG-200 Glyceryl Palmate oder PEG-120 Methyl Glucose Dioleate.

### Tenside:

Die Tenside, die in den erfindungsgemäßen Zubereitungen Verwendung finden, können anionische Tenside in Kombination mit amphoteren und/oder nichtionischen Tensiden sein.

Vorteilhaft zu verwendende anionische Tenside sind
Acylaminosäuren (und deren Salze), wie
1. Acylglutamate, beispielsweise Natriumacylglutamat, Di-TEA-palmitoylaspartat und Natrium Caprylic/ Capric Glutamat,
2. Acylpeptide, beispielsweise Palmitoyl-hydrolysiertes Milchprotein, Natrium Cocoylhydrolysiertes Soja Protein und Natrium-/ Kalium-Cocoyl-hydrolysiertes Kollagen,
3. Sarcosinate, beispielsweise Myristoyl Sarcosin, TEA-lauroyl Sarcosinat, Natriumlauroylsarcosinat und Natriumcocoylsarkosinat,
4. Taurate, beispielsweise Natriumlauroyltaurat und Natriummethylcocoyltaurat,
5. Acyllactylate, Lauroyllactylat, Caproyllactylat
6. Alaninate
Carbonsäuren und Derivate, wie
1. Carbonsäuren, beispielsweise Laurinsäure, Aluminiumstearat, Magnesiumalkanolat und Zinkundecylenat,
2. Ester-Carbonsäuren, beispielsweise Calciumstearoyllactylat, Laureth-6-Citrat und Natrium PEG-4-Lauramidcarboxylat,
3. Ether-Carbonsäuren, beispielsweise Natriumlaureth-13-Carboxylat und Natrium PEG-6-Cocamide Carboxylat,
Phosphorsäureester und Salze, wie beispielsweise DEA-Oleth-10-Phosphat und Dilaureth-Phosphat,
Sulfonsäuren und Salze, wie
1. Acyl-isethionate, z.B. Natrium-/ Ammoniumcocoyl-isethionat,
2. Alkylarylsulfonate,
3. Alkylsulfonate, beispielsweise Natriumcocosmonoglyceridsulfat, Natrium C12-14 Olefin-sulfonat, Natriumlaurylsulfoacetat und Magnesium PEG-3 Cocamidsulfat,
4. Sulfosuccinate, beispielsweise Dioctylnatriumsulfosuccinat, Dinatriumlaurethsulfosuccinat, Dinatriumlaurylsulfosuccinat, Dinatriumundecylenamido-MEA-Sulfosuccinat und PEG-5 Laurylcitrat Sulfosuccinat.
sowie
Schwefelsäureester, wie
1. Alkylethersulfat, beispielsweise Natrium-, Ammonium-, Magnesium-, MIPA-, TIPALaurethsulfat, Natriummyrethsulfat und Natrium C12-13-Parethsulfat,
2. Alkylsulfate, beispielsweise Natrium-, Ammonium- und TEA-Laurylsulfat.
Vorteilhaft zu verwendende amphotere Tenside sind
1. Acyl-/dialkylethylendiamin, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat,
2. N-Alkylaminosäuren, beispielsweise Aminopropylalkylglutamid, Alkylaminopropionsäure, Natriumalkylimidodipropionat und Lauroamphocarboxyglycinat.
Vorteilhaft zu verwendende nicht-ionische Tenside sind
1. Alkohole,
2. Alkanolamide, wie Cocamide MEA/ DEA/ MIPA,
3. Aminoxide, wie Cocoamidopropylaminoxid,
4. Ester, die durch Veresterung von Carbonsäuren mit Ethylenoxid, Glycerin, Sorbitan
5. oder anderen Alkoholen entstehen,
6. Ether, beispielsweise ethoxylierte/propoxylierte Alkohole, ethoxylierte/ propoxylierte Ester, ethoxylierte/ propoxylierte Glycerinester, ethoxylierte/ propoxylierte Cholesterine, ethoxylierte/ propoxylierte Triglyceridester, ethoxyliertes propoxyliertes Lanolin, ethoxylierte/ propoxylierte Polysiloxane, propoxylierte POE-Ether und Alkylpolyglycoside wie Laurylglucosid, Decylglycosid und Cocoglycosid.
7. Sucroseester, -Ether
8. Polyglycerinester, Diglycerinester, Monoglycerinester
9. Methylglucosester, Ester von Hydroxysäuren

### UV-Filter:

Es ist vorteilhaft im Sinne der vorliegenden Erfindungen, den Zubereitungen Sonnenschutzfilter zu zusetzen. Der hauptsächliche Zweck dieser Zubereitungen ist jedoch nicht der Schutz vor Sonnenlicht ist, sie enthalten aber dennoch einen Gehalt an UV-Schutzsubstanzen.

Es ist vorteilhaft im Sinne der vorliegenden Erfindung, wenn die verwendeten UVFiltersubstanzen wasserlöslich sind.

Erfindungsgemäße wasserlösliche UV-Filtersubstanzen sind z. B.:
1. Salze der 2-Phenylbenzimidazol-5-sulfonsäure, wie ihr Natrium-, Kalium- oder ihr Triethanolammonium-Salz, sowie die Sulfonsäure selbst;
2. Sulfonsäure-Derivate des 3-Benzylidencamphers, wie z. B. 4-(2-Oxo-3-bornylidenmethyl)-benzolsulfonsäure, 2-Methyl-5-(2-oxo-3-bornylidenmethyl)sulfonsäure und deren Salze.

Ferner können an Polymere gebundene öllösliche UV-Filter, wie z. B. Polysilicone-15, das auch unter dem Handelsnamen Parsol SLX erhältlich ist.

Es ist bevorzugt im Sinne der vorliegenden Erfindung beispielsweise Benzophenone-4 zu verwenden.

Die Gesamtmenge der Filtersubstanzen wird aus dem Bereich von 0,01 bis 30 Gew.-%, vorzugsweise 0,02 bis 1 Gew.-%, jeweils bezogen auf das Gesamtgewicht der Zubereitungen, gewählt.

### Lösungsmittel:

Die erfindungsgemäßen Zubereitungen können gegebenenfalls vorteilhaft Alkohole, Diole oder Polyole niedriger C-Zahl, sowie deren Ether, vorzugsweise Ethanol, Isopropanol, 1,2-Propandiol, Propylenglykol, Glycerin, Ethylenglykol, Ethylenglykolmonoethyl- odermonobutylether, Propylenglykolmonomethyl, -monoethyl- oder -monobutylether, Diethylenglykolmonomethyl-oder -monoethylether und analoge Produkte enthalten.

### Konservierungsmittel:

Zur Konservierung der vorliegenden Zubereitungen können die in der Kosmetik üblicherweise verwendeten Konservierungsmittel eingesetzt werden. Dazu zählen beispielsweise Parabene, wie Methylparaben, Propylparaben, Ethylparaben und Butylparaben. Wünschenswert ist aber auch der Einsatz von Konservierungsmitteln auf Säure-Basis, die in der Nahrungsmittelindustrie zum Einsatz kommen. Hierzu zählen beispielsweise Benzoesäure und/oder Salicylsäure und/oder ihre Salze. Da diese Konservierungsmittel ihre Wirkung in einem sauren pH-Wert-Bereich entfalten, einem pH-Bereich der für die menschliche Haut (pH-Wert der menschlichen Haut 5,4 bis 5,9, in einigen Hautarealen sogar unter pH-Wert = 5,0) vorteilhaft und für viele Bakterien unvorteilhaft ist, ist ihr Einsatz von großem Vorteil.

### pH-Wert Einstellung:

Die Einstellung des pH-Wertes kann auf die in der kosmetischen Industrie üblichen Art und Weise erfolgen. Bevorzugt ist jedoch der Einsatz von Zitronensäure und Natriumhydroxid um den erforderlichen pH-Wert einzustellen.

### Partikel:

Partikel im Sinne der vorliegenden Schrift sind Partikel aus allen organischen und anorganischen Feststoffen auf natürlicher und synthetischer Basis. Verwendet werden z.B. Kunststoffpartikel aus beispielsweise Viskose, Zellulose, Polypropylen, Polyester, Polyethylenterephthalat (PET), Polytetraflourethylen (PTFE), Aramid, Nylon, Kevla, Polyvinyldeivate, Polyurethane, Polystyrol, Celluloseester und/oder Polyethylen sowie alle Arten von Gesteinsmehl, gemahlene. Pflanzenbestandteile wie beispielsweise Nussschalen und Kerne Es können auch Mischungen verschiedener Partikel zum Einsatz kommen, die durch geeignete physikalische verfahren, wie z.B. Pressen pelletiert werden. Bevorzugt sind beispielsweise Unispheres^{®} der Firma Induchem oder Cosmospheres^{®} der Firma Pelletech.

Es ist dem Fachmann natürlich bekannt, dass kosmetische Zubereitungen zumeist nicht ohne die üblichen Hilfs- und Zusatzstoffe denkbar sind. Die erfindungsgemäßen kosmetischen Zubereitungen können dementsprechend ferner kosmetische Hilfsstoffe enthalten, wie sie üblicherweise in solchen Zubereitungen verwendet werden, beispielsweise Parfüme, Substanzen zum Verhindern des Schäumens, Farbstoffe, Pigmente, die färbende Wirkung haben, Emulgatoren, weichmachende, entzündungshemmende Substanzen, Insektenrepellentien, Bakterizide, Viruzide, Salze, antimikrobiell, proteolytisch oder keratolytisch wirksame Substanzen oder andere übliche Bestandteile einer kosmetischen Formulierung wie Schaumstabilisatoren und Elektrolyte.

### Beispiele: (Die Mengenangaben sind Aktiv-Gehalte)

| | **Beispiel Nr.** | **1** | **2** | **3** | **4** | **5** | **6** | **7** |
|---|---|---|---|---|---|---|---|---|
| A | Sodium Laureth Sulfate | 6,5 | 8,5 | 9,5 | 8,5 | 9,0 | 8,75 | 7,0 |
| B | Cocamidopropyl Betaine | 3,0 | 3,0 | | 3,2 | 3,5 | 2,9 | 2,8 |
| C | Sodium Myreth Sulfate | 3,0 | | | | | | 2,5 |
| D | Disodium Cocoyl Glutamate | | | | 0,5 | | | |
| E | Decyl Glucoside | | 1,0 | | | | | 1,0 |
| F | Coco Glucoside | | | 1,0 | | | | |
| G | Coco Betaine | | | 2,0 | | | | |
| H | Step-Polymer | 1,44 | 1,30 | 1,28 | 1,28 | 1,30 | 1,44 | 1,40 |
| I | PEG-7 Glyceryl Cocoate | 1,0 | 1,0 | 1,5 | 1,0 | 1,75 | 2,0 | 1,0 |
| J | PEG-40 Hydrogenated Castor Oil | 0,8 | 0,6 | 0,6 | 0,6 | 0,8 | 0,6 | 0,7 |
| K | Benzophenone-4 | | 0,05 | | 0,02 | 0,05 | | |
| | Glycerin | | | | | 1,0 | | |
| | Sodium Benzoate | 0,5 | 0,45 | 0,4 | 0,45 | 0,45 | 0,4 | 0,5 |
| | Sodium Salicylate | | | 0,2 | | 0,1 | 0,15 | |
| | Helianthus Annuus Seed Oil | | 0,1 | | | 0,1 | | 0,01 |
| | Cosmospheres® | 0,05 | 0,08 | | 0,15 | 0,08 | | |
| | Unispheres® | | | 0,1 | | | 0,2 | 0,15 |
| | Polyethylen particle | 0,1 | | | 0,2 | | | |
| | CI 42090 | | | 0,000 1 | | | 0,002 | |
| | CI 10316 | | | 0,002 | | | | |
| | Citric Acid | q.s. | q.s. | q.s. | q.s. | q.s. | q.s. | q.s. |
| | Sodium Hydroxide | q.s. | q.s. | q.s. | q.s. | q.s. | q.s. | q.s. |
| | Parfuem | 1,0 | 0,9 | 1,0 | 0,8 | 0,85 | 1,0 | 1,0 |
| | Aqua | ad 100 | ad 100 | ad 100 | ad 100 | ad 100 | ad 100 | ad 100 |
| | | | | | | | | |
| | **pH-Wert** | | **5,2** | | | | | |
| | **tan-delta** | | **0,16** | | | | | |
| | **Transmission [%]** | | **80,9** | | | | | |
| | **L/D** | | **0,6** | | | | | |
| | **Viskosität [mPa.s]** | | **2700** | | | | | |

| | **Beispiel Nr.** | **8** | **9** | **10** | **11** | **12** | **13** | **14** |
|---|---|---|---|---|---|---|---|---|
| A | Sodium Laureth Sulfate | 8,0 | 8,5 | 8,5 | 7,5 | 9,0 | 8,5 | 9,5 |
| B | Cocamidopropyl Betaine | 3,0 | 3,0 | | 3,0 | 3,0 | 2,9 | |
| C | Sodium Myreth Sulfate | | | | 2,0 | | | |
| D | Disodium Cocoyl Glutamate | 1,0 | | 0,5 | | | | |
| E | Decyl Glucoside | | | 1,0 | | | 0,5 | |
| F | Coco Glucoside | | 1,5 | | | 0,8 | 0,8 | 1,0 |
| G | Coco Betaine | | | 2,0 | | | | 2,0 |
| H | Step-Polymer | 1,44 | 1,28 | 1,28 | 1,32 | 1,40 | 1,60 | 1,28 |
| I | PEG-7 Glyceryl Cocoate | 1,2 | 1,0 | 1,0 | 1,0 | | 1,1 | 1,0 |
| J | PEG-40 Hydrogenated Castor Oil | 0,65 | 0,6 | 0,7 | 0,6 | 0,8 | 0,65 | 0,6 |
| K | Benzophenone-4 | | 0,05 | | 0,05 | 0,06 | 0,05 | 0,03 |
| | Glycerin | | | 0,5 | | | | |
| | Sodium Benzoate | 0,4 | 0,45 | 0,5 | 0,45 | 0,35 | 0,40 | 0,45 |
| | Sodium Salicylate | 0,1 | | | | 0,40 | 0,10 | |
| | Helianthus Annuus Seed Oil | 0,01 | | | 0,1 | | | 0,1 |
| | Cosmospheres® | 0,2 | | | 0,15 | | | |
| | Unispheres® | | 0,13 | | | | 0,11 | 0,08 |
| | Polyethylen particle | | | 0,3 | | 0,09 | 0,05 | |
| | CI 42090 | | | | | | 0,0001 | |
| | CI 15985 | | 0,0005 | | | | | |
| | Citric Acid | q.s | q.s. | q.s. | q.s. | q.s. | q.s. | q.s. |
| | Sodium Hydroxide | q.s. | q.s. | q.s. | q.s. | q.s. | q.s. | q.s. |
| | Parfuem | 1,0 | 0,8 | 0,9 | 1,0 | 1,1 | 0,85 | 1,0 |
| | Aqua | ad 100 | ad 100 | ad 100 | ad 100 | ad 100 | ad 100 | ad 100 |
| | | | | | | | | |
| | **pH-Wert** | | | | **5,2** | | | **5,2** |
| | **tan-delta** | | | | **0,19** | | | **0,17** |
| | **Transmission [%]** | | | | **84,1** | | | **84,0** |
| | **L/D** | | | | **0,5** | | | **0,6** |
| | **Viskosität [mPa.s]** | | | | **2850** | | | **2300** |

| | **Beispiel Nr.** | **15** | **16** | **17** | **18** | **19** | **20** | |
|---|---|---|---|---|---|---|---|---|
| A | Sodium Laureth Sulfate | 9,0 | 9,0 | 8,5 | 7,0 | 605 | 7,5 | |
| B | Cocamidopropyl Betaine | 3,0 | 3,0 | 3,1 | 3,0 | 3,2 | 3,0 | |
| C | Sodium Myreth Sulfate | | | | 2,0 | 3,0 | | |
| D | Disodium Cocoyl Glutamate | | | 0,5 | | | 1,0 | |
| E | Decyl Glucoside | | 1,0 | 0,2 | | | 0,3 | |
| F | Coco Glucoside | | | 0,3 | 0,5 | 0,6 | 0,4 | |
| H | Step-Polymer | 1,28 | 1,44 | 1,45 | 1,40 | 1,35 | 1,44 | |
| I | PEG-7 Glyceryl Cocoate | 1,0 | 1,5 | 1,2 | 1,0 | 1,1 | | |
| J | PEG-40 Hydrogenated Castor Oil | 0,6 | 0,6 | 0,8 | 0,7 | 0,65 | 0,6 | |
| K | Benzophenone-4 | 0,05 | 0,05 | | 0,04 | | | |
| | Glycerin | | | | | 1,0 | | |
| | Sodium Benzoate | 0,45 | 0,45 | 0,40 | 0,35 | 0,50 | 0,4 | |
| | Sodium Salicylate | | | 0,10 | 0,15 | | 0,1 | |
| | Helianthus Annuus Seed Oil | 0,1 | | | 0,01 | | | |
| | Cosmospheres® | | 0,08 | | | 0,07 | | |
| | Unispheres® | 0,1 | | 0,15 | | | 0,13 | |
| | Polyethylen particle | | | | 0,1 | | | |
| | CI 10316 | | | 0,002 | | | | |
| | CI 16035 | | | | | 0,0004 | | |
| | Citric Acid | q.s. | q.s. | q.s. | q.s. | q.s. | q.s. | |
| | Sodium Hydroxide | q.s. | q.s. | q.s. | q.s. | q.s. | q.s. | |
| | Parfuem | 1,0 | 0,9 | 0,85 | 1,0 | 1,0 | 1,3 | |
| | Aqua | ad 100 | ad 100 | ad 100 | ad 100 | ad 100 | ad 100 | |
| | | | | | | | | |
| | **pH-Wert** | **5,3** | | | | | | |
| | **tan-delta** | **0,25** | | | | | | |
| | **Transmission [%]** | **83,3** | | | | | | |
| | **L/D** | **0,5** | | | | | | |
| | **Viskosität [mPa.s]** | **2400** | | | | | | |

| **Rohstoff** | **Handelsname** | | | | | | | |
|---|---|---|---|---|---|---|---|---|
| A | Texapon® N 70, BASF | | | | | | | |
| B | Tego-Betain® F 50, Degussa | | | | | | | |
| C | Texapon® K 14 S Spezial, 70%, BASF | | | | | | | |
| D | Amisoft® ECS 22 SB (ph10), Ajinomoto | | | | | | | |
| E | Plantacare® 2000 UP, BASF | | | | | | | |
| F | Plantacare® 818 UP, BASF | | | | | | | |
| G | Dehyton® AB 30, BASF | | | | | | | |
| H | Carbopol® Aqua SF-2 Polymer | | | | | | | |
| I | Cetiol® HE, BASF | | | | | | | |
| J | Eumulgin® HRE 40, BASF | | | | | | | |
| K | Uvasorb® S 5, CoastSouthwest | | | | | | | |

## Patentansprüche

1. Transparente Reinigungszubereitungen mit suspendierten Partikeln, einem pH-Wert von 4,5 bis 5,5 und einer Viskosität von 2000 mPa·s bis 5000 mPa·s, **dadurch gekennzeichnet, dass** wenigstens ein Step-Polymer und anionische Tenside und amphotere und optional nichtionische Tenside enthalten sind, wobei der Gehalt an anionischen Tensiden unter 10 Gew.-% liegt, bezogen auf die Gesamtmasse der Zubereitung.

2. Zubereitung nach Patentanspruch 1, **dadurch gekennzeichnet, dass** der Gehalt an wenigstens einem Step-Polymer höchstens 2,5 Gew.-%, bevorzugt höchstens 2,0 Gew.-%, insbesondere bevorzugt zwischen 1,0 und 1,6 Gew.-%, bezogen auf den Aktivgehalt und das Gesamtgewicht der Zubereitung, beträgt.

3. Zubereitung nach Patentanspruch 1, **dadurch gekennzeichnet, dass** der Gehalt an gelbildenden Polymeren einschließlich von wenigstens einem Step-Polymer höchstens 5,0 Gew.-%, bevorzugt höchstens 2,5 Gew.-%, insbesondere bevorzugt 1,6 Gew.-%, bezogen auf den Aktivgehalt und das Gesamtgewicht der Zubereitung, beträgt.

4. Zubereitung nach einem der vorangehenden Patentansprüche, **dadurch gekennzeichnet, dass** das Step-Polymer ein Acrylates Crosspolymer-4 ist.

5. Zubereitung nach einem der vorangehenden Patentansprüche, **dadurch gekennzeichnet, dass** der tan-delta der Zubereitung kleiner 0,6 ist.

6. Zubereitung nach einem der vorangehenden Patentansprüche, **dadurch gekennzeichnet, dass** die Transmission größer 80% ist.

7. Zubereitung nach einem der vorangehenden Patentansprüche, **dadurch gekennzeichnet, dass** eine besondere Milde der Zubereitung erreicht wird, **gekennzeichnet durch** L/D-Werte ≥ 0,5.

8. Zubereitung nach einem der vorangehenden Patentansprüche, **dadurch gekennzeichnet, dass** die Zubereitung frei von Xanthangummi ist.

9. Verfahren zur Herstellung der Zubereitungen nach Anspruch 1, umfassend folgende Schritte:
- Wasser vorlegen (optional Farbstoff einarbeiten)
- Polymer, einschließlich Step-Polymer, in die Wasserphase geben,
- anionisches Tensid zugeben (falls in der Rezeptur vorgesehen)
- Nicht-ionisches Tensid zugeben (falls in der Rezeptur vorgesehen)
- amphoteres Tensid zugeben (falls in der Rezeptur vorgesehen)
- Neutralisation mit NaOH auf pH: 6,5-6,8
- Lichtschutzphase zugeben (falls in der Rezeptur vorgesehen)
- Glycerin zugeben (falls in der Rezeptur vorgesehen)
- Parfümphase zugeben (falls in der Rezeptur vorgesehen)
- Konservierungsmittel-Phase zugeben (falls in der Rezeptur vorgesehen)
- pH-Wert mit Citonensäure (50%ig) auf 4,5-5,5 einstellen
- feste Partikel und/oder Peelingkörper einarbeiten mit möglichst geringer Scherung

10. Verwendung einer Zubereitung nach wenigstens einem der Patentansprüche 1 bis 6 zur Reinigung der Haut des Menschen.

11. Verwendung einer Zubereitung nach wenigstens einem der Patentansprüche 1 bis 7 zur Reinigung der Haare des Menschen.
